Europäisches Patentamt

(19) European Patent Office

Office européen des brevets

(11) Publication number : **0 398 621 B1**

## (12) EUROPEAN PATENT SPECIFICATION

(45) Date of publication of patent specification :
**21.12.94 Bulletin 94/51**

(51) Int. Cl.⁵ : **G01N 33/573**

(21) Application number : **90305189.4**

(22) Date of filing : **15.05.90**

(54) **Immunoassay of elastase-1.**

(30) Priority : **18.05.89 JP 126423/89**

(43) Date of publication of application :
**22.11.90 Bulletin 90/47**

(45) Publication of the grant of the patent :
**21.12.94 Bulletin 94/51**

(84) Designated Contracting States :
**AT BE CH DE DK ES FR GB GR IT LI LU NL SE**

(56) References cited :
EP-A- 0 038 935
EP-A- 0 080 279
US-A- 4 458 013
FEBS LETTERS vol. 67, no. 2, August 1976,pages 156-160, Amsterdam, NL; A. KOJ et al.: "Inactivation of some pancreaticand leucocyte elastases by peptide chloromethyl ketones and alkyl isocyanates"
PATENT ABSTRACTS OF JAPAN vol. 10, no. 296 (C-377)(2352), 8 October 1986; & JP - A - 61111688 (KIRIN BREWERY CO. LTD.) 29.05.1986

(56) References cited :
CLINICAL CHEMISTRY vol. 26, no. 13, 1980, pages 1854-1859, Winston, Winston, US; B.K. CHOE et al.: "Double-antibody immunoenzyme assay for human prostatic acid phosphatase"

(73) Proprietor : **SHIONOGI SEIYAKU KABUSHIKI KAISHA trading under the name of SHIONOGI & CO. LTD.**
**1-8, Doshomachi 3-chome,**
**Chuo-ku**
**Osaka 541 (JP)**

(72) Inventor : **Yoshida, Nobuo**
**5-8, Koshiensanban-cho**
**Nishinomiya-shi, Hyogo (JP)**
Inventor : **Inouye, Ken**
**1296, Zenkai, Ikawadani-cho**
**Nishi-ku**
**Kobe-shi, Hyogo (JP)**
Inventor : **Kono, Masao**
**3-25-28, Ayukawa**
**Ibaraki-shi, Osaka (JP)**

(74) Representative : **Allard, Susan Joyce et al**
**BOULT, WADE & TENNANT**
**27 Furnival Street**
**London EC4A 1PQ (GB)**

EP 0 398 621 B1

## Description

This invention relates to an immunoassay of elastase-1 which is a proteolytic enzyme secreted from the pancreas and contained in body fluids. This immunoassay is useful for the diagnosis of pancreatic diseases. Further, this invention relates to the human elastase-1 treated with a compound of the formula:

$$(CH_3)_3COCO[NHCH(CH_3)CO]_3CH_2Cl$$

(hereinafter abbreviated as Boc-TACK), which is useful for said immunoassay.

The incidence of pancreatic diseases has been showing an increasing tendency in recent years due to various reasons and this type of disease is now serious in the clinical field. However, since the pancreas is situated in the deepest part of the abdominal cavity, it is difficult to detect said diseases and there has been a demand for the development of a simple and accurate method of diagnosis therefor.

As biochemical methods of diagnosis for pancreatic diseases, a method is known in which amylase in the patient's body fluids (mainly blood and urine, and also pancreatic juice, pleural dropsy and abdominal dropsy) is measured. However, the blood amylase measured by this method shows the total amount of two amylases, one originating from the salivary gland and the other from the pancreas. Therefore, this method of measuring amylase is not regarded as a specific diagnostic method for pancreatic diseases. Besides, the period in which amylase shows high values after onset of the pancreatic diseases is short, and when this period is over, it is impossible to make the diagnosis. For this reason, there has been a demand for the development of some other method for diagnosis of pancreatic diseases by measuring pancreatic enzymes.

Human pancreas or pancreatic juice contains, in addition to amylase, elastase which is an enzyme decomposing elastin, a fibrous protein. There are two types of elastase. One is elastase-1 (molecular weight 29,000 - 33,000) with acid charge, and the other is elastase-2 (molecular weight 25,000 - 26,000) with basic charge. Elastase-1 activates elastase-2. These elastases are different from each other in terms of enzymology, protein chemistry and immunology. The measurement of elastase-1 is useful in the diagnosis and observation of the subsequent development of pancreatic diseases, especially acute pancreatitis, chronic and recurrent pancreatitis, and cancer of the pancreas. Serum elastase-1 is known to increase remarkably in acute pancreatitis. It is also known to increase more remarkably that other pancreatic enzymes in cancer of the pancreas.

Attempts have hitherto been made to measure elastase-1 based upon its enzymatic activity. However, it has been difficult to measure elastase-1 by this method for the following reasons: The amount of elastase contained in blood is very small. In addition, the blood contains a large amount of elastase inhibitors, $\alpha_1$-antitrypsin and $\alpha_2$-macroglobulin, and these inhibit elastase from showing its activity.

As shown above, elastase is present in blood, being bound to inhibitors, $\alpha_1$-antitrypsin and $\alpha_2$-macroglobulin. $\alpha_1$-antitrypsin is bound to the active site of elastase, but the structure itself of elastase remains unchanged. Therefore, it has an immunological activity, and can take part in antigen-antibody reaction. On the other hand, the inhibition by $\alpha_2$-macroglobulin is done in a form which masks elastase. As a result, it becomes impossible for antibodies to recognize the structure of elastase and, consequently, it becomes impossible to measure elastase even by immunological procedures. Therefore, attempts were made to measure, by an immunological method, the elastase bound to $\alpha_1$-antitrypsin in the blood. In immunoassay, however, the immunological activity of the labelled elastase varies depending on the amount of elastase inhibitor contained in the serum specimen. Consequently, it was difficult to make accurate quantitative determination of elastase.

Meanwhile, it was found that, when the labelled elastase was pretreated with diisopropyl fluoropbosphate (DFP), a synthetic inhibitor of elastase, it became impossible for the elastase inhibitor in the blood to bind to the labelled elastase. Consequently, a method for the immunoassay of elastase-1 with the use of the aforesaid DFP-treated elastase-1 was developed (Japanese Patent Publication No. 25183/1984). There was also developed a method (Japanese Patent Unexamined Publication No. 73152/1988 and US-A-4458013) in which labelled elastase-1 treated with DFP, phenylmethanesulfonyl fluoride (PMSF), p-chloromercuribenzoate (PCMB) or $\alpha_1$-antitrypsin and anti-elastase monoclonal antibody are used.

As synthetic inhibitors which inhibit the elastase from being bound to the inhibitors in the blood while maintaining the immunological activity of the elastase, there are known DFP, PMSF and PCMB. On the other band, Boc-TACK is known to inhibit the enzymatic activity of porcine elastase (FEBS LETTERS Vol. 67, 156-160 (1976)). However, it has not been known that Boc-TACK inhibits the enzymatic activity of human elastase. Nor has it been known that Boc-TACK inhibits the elastase-1 from being bound to $\alpha_1$-antitrypsin.

In the conventional method of immunoassay, elastase inhibitors such as $\alpha_1$-antitrypsin and $\alpha_2$-macroglobulin in the serum bind to labelled elastase-1, thereby inhibiting the immunological activity of the labelled elastase-1. Therefore, it was impossible to make accurate quantitative determination of blood elastase-1.

For the prevention of the binding of $\alpha_1$-antitrypsin and $\alpha_2$-macroglobulin to elastase-1, a method is known in which the active group of labelled elastase-1 is blocked by DFP, PMSF and PCMB (mentioned above). However, of these synthetic inhibitors, PMSF has a weak capability of inhibiting the binding of $\alpha_1$-antitrypsin and

EP 0 398 621 B1

$\alpha_2$-macroglobulin to elastase-1.

Besides, DFP and PMSF are toxic since they contain fluorine and therefore generate hydrogen fluoride when binding to elastase-1. Furthermore, DFP causes disturbance of the optic nerve. Therefore, care must be taken when using these materials.

Meanwhile, PCMB contains mercury, so its use may bring about environmental pollution.

For these reasons, there has been a demand for a safe synthetic inhibitor free from toxic substances such as fluorine and mercury which completely inhibits the binding of inhibitors in the blood to elastase-1 without disturbing the immunological activity of the elastase-1 and which can take the place of PMSF and DFP.

This invention provides an immunoassay of elastase-1, which comprises subjecting (a) labelled elastase-1 treated with Boc-TACK and (b) elastase-1 to be measured to competitive reaction with anti-elastase-1 antibody, wherein the Boc-TACK denotes

$$(CH_3)_3COCO[NHCH(CH_3)CO]_3CH_2Cl.$$

The synthetic inhibitor Boc-TACK almost completely inhibits the binding of $\alpha_1$-antitrypsin and $\alpha_2$-macroglobulin to elastase-1 without disturbing the immunological activity of the elastase-1 and realizes a simple and accurate immunoassay of elastase-1. Further, since the Boc-TACK generates no toxic hydrogen fluoride in the treatment of elastase-1 therewith and contains no harmful mercury, it is much safer to use than conventional inhibitors such as PCMB, PMSF and DFP.

This invention further provides the Boc-TACK-treated human elastase-1, which essentially does not react with $\alpha_1$-antitrypsin or with $\alpha_2$-macroglobulin.

Fig. 1 shows the results of the elution of $^{125}$I-labelled E1(a) not treated with Boc-TACK and Boc-TACK-treated $^{125}$I-labelled E1(b), both in human serum, from Ultrogel AcA 44 (1.5 x 55 cm) with the use of 50 mM phosphate buffer (pH 7.1).

Fig. 2 shows the effect of $\alpha_1$-antitrypsin and $\alpha_2$-macroglobulin on the rate of the binding of anti-E1 serum with $^{125}$I E1 not treated or treated with PMSF or Boc-TACK.

Fig. 3 gives standard curve of E1 and dilution curve of human serum.

Fig. 4 illustrates the effect of Boc-TACK-treated E1 on standard curve.

Fig. 5 shows cross reactivity of E1 and E1 complexes containing $\alpha_1$-antitrypsin or $\alpha_2$-macroglobulin.

Fig. 6 shows cross reactivity with several animal sera.

In order to search for a synthetic inhibitor which is superior to PMSF and DFP and which almost completely inhibits the binding of $\alpha_1$-antitrypsin and $\alpha_2$-macroglobulin to elastase-1 without disturbing the immunological activity of the elastase-1 so as to establish a better and more accurate method for the measurement of elastase-1, the inventors made strenuous efforts and studies and, as a result, have completed a method for the immunoassay of elastase-1 which involves the use of Boc-TACK, a synthetic inhibitor.

The term Boc-TACK as used in this specification is an abbreviation (Boc Tri-Alanyl Chloromethyl Ketone) of N$_\alpha$-[N-(tert-butoxycarbonyl)alanyl]-N-(3-chloro-1-methyl-2-oxopropyl)alanineamide having the following formula:

$$(CH_3)_3COCO[NHCH(CH_3)CO]_3CH_2Cl$$

wherein the alanine residues are preferably of L configuration.

The human elastase-1 (EC 3.4.21.11) used in this invention may be obtained by purifying elastase-1 obtained from the human pancreatic juice in accordance with conventional methods such as the method of Feinstein et al. (Eur. J. Biochem 43, 569 (1974)). A label for the human elastase-1 obtained may be chosen from among conventional labelling agents such as radioisotope, enzyme, fluorescent substance and luminescent substance. For labelling by radioactive iodine, the chloramine T method, lactoperoxidase method or Bolton-Hunter method may be employed. As for radioactive iodine, $^{125}$I and $^{131}$I are used. As labelling agents, there are used $\beta$-galactosidase, horseradish peroxidase, glucose oxidase, alkaline phosphatase, glucose-6-phosphatase, dehydrogenase, etc. As fluorescent substances, there are used substances of fluorescein type such as fluorescein isothiocyanate and materials of rhodamine type such as tetramethylrhodamine isothiocyanate. In this invention, it is especially preferable to label with $^{125}$I by the chloramine T method.

The labelled human elastase-1 obtained is processed into Boc-TACK-bound labelled human elastase-1 by treatment with Boc-TACK. This process may be performed, for example, by adding labelled human elastase-1 into a suitable buffer solution, such as phosphate buffer, containing Boc-TACK and then by allowing it to stand overnight so as to cause Boc-TACK to be bound to the labelled human elastase-1. Since the binding of Boc-TACK to elastase-1 does not produce any hydrogen fluoride, this is a safe process.

As anti-elastase-1 antibody, antisera may be obtained from mammals such as rabbits, goats, chickens and guinea pigs immunized with human elastase-1 together with a suitable adjuvant such as Freund's complete adjuvant. As anti-elastase-1 antibody, a monoclonal antibody may also be used.

The immunoassay of elastase-1 of this invention involves the labelled human elastase-1 treated with Boc-TACK as above and the elastase-1 to be measured, human elastase-1 for example, being subjected to com-

3

petitive reaction with above-mentioned anti-elastase-1 antibody.

After the competitive reaction, the labelled human elastase-1 to which anti-elastase-1 antibody has bound and the free labelled human elastase-1 are separated by a conventional method to obtain each fraction thereof, and then the activity of the labelling agent in either or both of said fractions is measured. Generally, it is enough to measure the activity in the antibody-bound fraction.

The aforesaid separation of the fractions may be performed by known methods such as solid phase methods, sandwich methods and double antibody methods. However, in Examples of this invention, a double antibody method is used for the separation. As for the second antibody used in the double antibody method, immuno-beads-goat anti-rabbit IgG antibody may be used where rabbit serum is used as an anti-elastase-1 antibody.

The values obtained from the measurement of the labelling agent in the above fractions are compared with the standard curve obtained with the standard elastase-1 having known concentrations, from which the amount of the elastase-1 to be measured is calculated.

This invention further relates to the Boc-TACK-treated human elastase-1 obtained as above. As shown in the Examples found herein, the Boc-TACK-treated human elastase-1 of this invention essentially does not react with $\alpha_1$-antitrypsin or with $\alpha_2$-macroglobulin. Besides, no toxic hydrogen fluoride is generated in treatment with Boc-TACK. Nor does Boc-TACK contain any harmful mercury.

Furthermore, DFP and PMSF bind to all serine residues, whereas Boc-TACK binds specifically to the active center of elastase-1. Thus, the immunological activity of elastase-1 is little affected by Boc-TACK.

Therefore, Boc-TACK is suitable for use in a measurement system where $\alpha_1$-antitrypsin or $\alpha_2$-macroglobulin is present. It becomes even more useful when it is labeled beforehand with an enzyme, radioisotope or fluorescent substance.

## Example 1

### Synthesis of elastase inhibitor Boc-TACK:

### $(CH_3)_3COCO[NHCH(CH_3)CO]_3CH_2Cl$ (I)

The above-mentioned tripeptide derivative was prepared according to the following procedures. Hereinafter, Boc-TACK is referred to as Boc-Ala-Ala-Ala-CH$_2$Cl.

H-Ala-OBzl
$\downarrow$ Boc-Ala-OSu
Boc-Ala-Ala-OBzl (II)
$\downarrow$ 20%·TFA/CH$_2$Cl$_2$
$\downarrow$ Boc-Ala-OSu, DIEA
Boc-Ala-Ala-Ala-OBzl (III)
$\downarrow$ H$_2$/Pd
Boc-Ala-Ala-Ala-OH (IV)
$\downarrow$ ClCO$_2$CH$_2$CH(CH$_3$)$_2$, N-methyl morpholine
$\downarrow$ CH$_2$N$_2$/(C$_2$H$_5$)$_2$O
Boc-Ala-Ala-Ala-CHN$_2$ (V)
$\downarrow$ 1M HCl/AcOH
H-Ala-Ala-Ala-CH$_2$Cl (VI)
$\downarrow$ (Boc)$_2$O/DMF
Boc-Ala-Ala-Ala-CH$_2$Cl (I)

### Boc-Ala-Ala-OBzl (II)

In 10 ml of DMF (N,N-dimethylformamide) is dissolved H-Ala-OBzl p-toluenesulfonate (2.0 g, 5.7 mmol), which is then cooled with ice. To this are added equimolar DIEA (N,N-diisopropylethylamine) and Boc-Ala-OSu (HOSu: N-hydroxysuccinimide) (1.96 g, 6.8 mmol). The mixture is allowed to undergo reaction for 20 hours at 25°C, after which the solvent is evaporated under reduced pressure. The residue is dissolved in ethyl acetate, which is then washed with 1 M HCl, 1 M NaHCO$_3$ and H$_2$O in the order mentioned. After that, the ethyl acetate layer is dried with MgSO$_4$. The solvent is then evaporated, and the crystals obtained are purified on a silica gel column (Kieselgel H, 50 g, CHCl$_3$ : MeOH = 99 : 1), followed by recrystallization from ether-petroleum ether, whereby 1.85 g (93.0%) of compound II is obtained.
m.p. 65 - 67°C

$[\alpha]_D^{24}$ - 26.9 ± 0.7° (c = 1.0, DMF)


Boc-Ala-Ala-Ala-OBzl (III)

To the compound II (1.8 g, 5.1 mmol) obtained above is added 20% TFA (trifluoroacetic acid)/$CH_2Cl_2$, which is then allowed to undergo reaction for 30 minutes at 25°C. The solvent is evaporated, and the residue is dissolved in 20 ml of DMF. The solution is cooled with ice, and is then neutralized with DIEA. To this is added Boc-Ala-OSu (1.65 g, 6.2 mmol), and the mixture is allowed to undergo reaction for 20 hours at 25°C. The reaction solution is treated in the same manner as in the above-mentioned compound II. The crude product obtained is purified with silica gel (Kieselgel H, 50 g, $CHCl_3$ : MeOH = 98 : 2), followed by recrystallization from ether to give 2.0 g (93%) of compound III, m.p. 140 - 141°C.
$[\alpha]_D^{24}$ - 30.4 ± 0.7° (c = 1.0, DMF)


Boc-Ala-Ala-Ala-OH (IV)

Compound III (2.0 g, 4.75 mmol) obtained as above is subjected, by a conventional method, to catalytic reduction in methanol for 5 hours with palladium black (Pd black) as catalyst ($H_2$ consumption ca. 100 ml). The solvent is evaporated and the residue obtained is recrystallized from methanol-ether to give 1.56 g (99%) of the compound IV, m.p. 200 - 201°C (decomp.).
$[\alpha]_D^{24}$ - 20.5 ± 0.6° (c = 1.0, DMF)


Boc-Ala-Ala-Ala-$CHN_2$ (V)

Compound IV (1.09 g, 3.3 mmol) obtained as above is dissolved in 20 ml of anhydrous THF (tetrahydrofuran), which is then cooled to -10°C. To this are added 0.36 ml (3.3 mmol) of N-methylmorpholine and then 0.45 ml (3.3 mmol) of isobutyl chloroformate and the mixture is stirred for 30 minutes at the same temperature. To this is added a solution of diazomethane in ether and the reaction is allowed to take place for 30 minutes. After confirming that the yellow color of diazomethane is still present, the solvent is evaporated under reduced pressure. To the residue is added ether and the resulting gelatinous precipitates are collected by filtration. The product obtained is then purified using a silica gel column (Kieselgel H, 50 g, $CHCl_3$: MeOH = 98 : 2), followed by recrystallization from ether, whereby 0.60 g (51%) of the compound V is obtained as yellow needles, m.p. 165°C (decomp.). IR: 2108 (-$CHN_2$), 1676 (>C = O) $cm^{-1}$
$^1$H NMR: 6.03 ppm (-CO-$CHN_2$)


H-Ala-Ala-Ala-$CH_2Cl$·HCl (VI)

Compound V (500 mg, 1.4 mmol) obtained as above is dissolved in 5 ml of 1 M HCl/AcOH. The solution is kept at 0°C for 30 minutes, and then at 25°C for 60 minutes, followed by evaporation to dryness under reduced pressure. The residue is subjected to partition chromatography on a Sephadex LH-20 column (4 x 35 cm), with 1-BuOH - AcOH - EtOH - $H_2O$ (2:2:1:1) as solvent. Fractions containing the desired product are collected and the solvent is evaporated to give 200 mg of crude product. The thin-layer chromatography (Kieselgel H, AcOEt -AcOH - $H_2O$ = 4:1:1, ninhydrin coloration) of the crude product shows two spots.


Boc-Ala-Ala-Ala-$CH_2Cl$ (I)

Compound VI (ca. 100 mg) obtained as above is dissolved in 2 ml of DMF, which is then neutralized with DIEA. To this is added (Boc)$_2$O (100 mg, ca. 0.5 mmol) and the mixture is allowed to undergo reaction for 30 minutes at 25°C. Then, the solvent is evaporated, and the residue is purified on a silica gel column (Kieselgel H, 20 g, $CHCl_3$ : MeOH = 98:2). Fractions containing the desired product are collected and the solvent is evaporated, followed by crystallization from ether to give 51 mg of the objective compound I, m.p. 205°C (decomp.).
$[\alpha]_D^{23}$ - 90.8 ± 2.6° (c = 0.5, MeOH)
$^1$H NMR: 4.35 ppm (-CO-$CH_2Cl$)

Example 2

Radioimmunoassay of human serum elastase-1 with the use of Boc-TACK-bound $^{125}$I-labelled elastase-1

For pancreatic elastase-1 in blood (hereinafter abbreviated as E1) measured by radioimmunoassay (competitive method), labelled antigen ($^{125}$I-labelled E1) will bind to inhibitors, $\alpha_1$-antitrypsin and $\alpha_2$-macroglobulin in the blood, thereby inhibiting the antigen-antibody reaction. As a result, it becomes impossible to accurately measure serum E1. To prevent this, a method is known in which the active group (binding site) of $^{125}$I-labelled E1 is blocked with PMSF, DFP, etc. (as mentioned above).

In the following Examples, $^{125}$I-labelled E1 treated with Boc-TACK does not bind to the serum inhibitors and all of it can take part in antigen-antibody reaction. This was confirmed by gel filtration and also by immunological procedures. Thus, Boc-TACK proves to be useful for the radioimmunoassay of human serum E1.

I. Reagents and method

(1) Preparation of elastase

To $4\ell$ of human pancreatic juice is added 2.8 kg (70% saturation) of ammonium sulfate, which is then allowed to stand at 4°C for 1 - 2 days. The precipitate is collected by centrifugation, and is suspended in 200 ml of buffer solution 1 (50 mM sodium acetate and 10 mM $CaCl_2$, pH 6.5), and then dialyzed for 2 days against the same buffer solution (outer solution $5\ell$, replaced 4 times). To lower the conductivity of the dialysis inner solution (775 ml), buffer solution 1 is added to bring the volume finally to $2.0\ell$ (0.95 mho, pH 6.45). The mixture is loaded on a SP-Sephadex (registered Trade Mark) C-25 column (2.5 x 41.0 cm) buffered beforehand with buffer solution 1. It is then washed with buffer solution 1 equivalent to 10 times the column volume, after which it is eluted with buffer solution 1 equivalent to 15 times the volume of the resin with a linear concentration gradient of $0 \rightarrow 0.4$ M·NaCl. In the pass-through fraction were seen an activity for decomposing elastin and an activity for decomposing succinyl-tri-alanine-p-nitroanilide (hereinafter abbreviated to $SA_3PNA$), both brought about by elastase. However, in this fraction there was also found some trypsin and chymotrypsin as well as elastase. Therefore, this fraction is collected (1295 ml), and to this is added 903 g (70% saturation) of ammonium sulfate. The mixture is allowed to stand at 4°C for 3 days. After that, the precipitate is collected, and is then dissolved in about 40 ml of buffer solution 1. This elastase fraction is dialyzed for 4 days against buffer solution 1 (outer solution $5\ell$, replaced twice). After bringing its volume to 82 ml, it is charged on a Sepharose (Registered Trade Mark) 4B-ε Aminoceproyl-(Ala)$_3$ column (1.5 x 24.0 cm). The trypsin and chymotrypsin mixed therein passed through completely, but elastase showed was adsorbed slightly. However, the elastase fraction still contains a small quantity of trypsin and chymotrypsin. To remove these, elastase fractions are collected (147.0 ml), and are then concentrated by ultrafiltration (Amicon, YM-10) to a volume of 45 ml. It is then dialyzed for two days (outer solution $5\ell$, replaced twice) against buffer solution 2 (10 mM Tris-HCl and 2 mM $CaCl_2$, pH 7.5). The volume finally comes to 57 ml, which is charged on a sepharose (Registered Trade Mark) 4B-ε Aminocaproyl (Ala), column (1.5 x 24.0 cm) buffered beforehand with buffer solution 2. Elastase absorbed slightly.

However, most of it was eluted in the vicinity of the passing fraction Chymotrypsin came out before elastase and trypsin after elastase. Thus, the trypsin and chymotrypsin mixed in elastase were removed almost completely. There was obtained finally 52.8 mg of elastase.

(2) Properties of elastase

(A) Purity

The uniformity of the obtained elastase was confirmed by each of SDS-PAGE (15% acrylamide, 1 mm gel), pH 9.4 gel electrophoresis (7.5% acrylamide, 1 mm gel), and pH 4.0 gel electrophoresis (7.5% acrylamide, 1 mm gel).

(B) Molecular weight

When calculated from the transfer rate in the aforesaid SDS-PAGE with the use of electrophoresis calibration kit made by Pharmacia, the molecular weight was 31500. This was larger than the molecular weight of 25590 (Japanese Patent Unexamined Publication No. 111688/1986) estimated of elastase-1 based on cDNA. Since its amino acid composition is nearly the same as that of the elastase-1 described in Japanese Patent Unexamined Publication No. 111688/1986, it is presumed that the accurate molecular weight was not reflected in the aforesaid measurement of molecular weight by SDS-PAGE due to the peculiarity of elastase molecule.

(C) Analysis of amino acids

The obtained elastase was desalted with HPLC (Brownlee, Aquapore RP-300 column). The resulting product was hydrolyzed for 24 hours at 110°C with the use of 4 M methanesulfonic acid containing 0.2% 3-(2-aminoethyl)indole. The analysis of amino acids was performed with Hitachi Amino Acids Analyzer (Model 835), the results of which are shown in Table 1. The amino acid composition obtained in the present measurement was nearly the same as that of elastase-1 estimated from cDNA.

Table 1

| Amino acids | Results | Reference[*1] |
|---|---|---|
| Asp | 22.0 | 21 |
| Thr | 12.8 | 13 |
| Ser | 19.8 | 19 |
| Glu | 19.9 | 19 |
| Pro | 15.9 | 16 |
| Gly | 24.8 | 25 |
| Ala | 15.0 | 14 |
| Cys/2 | 9.6 | 10 |
| Val | 18.4 | 22 |
| Met | 1.1 | 1 |
| Ile | 11.1 | 13 |
| Leu | 17.0 | 18 |
| Tyr | 9.0 | 9 |
| Phe | 7.8 | 7 |
| Lys | 7.2 | 9 |
| His | 5.8 | 7 |
| Arg | 9.0 | 8 |
| Trp | 8.6 | 11 |
| Total | | 242 |

[*1]  See Japanese Patent Unexamined Publication No. 111688/1986.

(D) Enzymatic activity

The measurements were made as follows: Elastin-decomposing activity, by the method of W. Ardelt et al. (Anal. Biochem 34, 180, (1970); casein-decomposing activity, by the method of Nomoto et al. (Riken Report 34.6 381, (1958)); esterase-activity on BTEE (benzoyl-tyrosine-ethyl ester) and TAME (tosyl-arginine-methyl ester), by the method of B.C.W. Hammel (Can. J. Biochem. Physiol 37, 1393 (1959)); and activity on SA$_3$PNA, by the method of J. Bieth et al. (Biochem. Med. 11, 350, (1974)). The results are shown in Table 2. For comparison, activities of porcine elastase made by Sigma, and bovine chymotrypsin and bovine trypsin both made by Worthington are also shown. As compared with porcine elastase, the human elastase obtained in the present study was about the same in casein-decomposing activity, but considerably weaker in elastin-decomposing activity, the value being about 1/10 - 1/20 of that of porcine elastase. As for SA$_3$PNA-decomposing activity, the human elastase was weaker to some extent, but it still showed strong activity. Such a tendency is generally known of elastase-1. The human elastase obtained in the present study is presumed to be elastase-1 not only from its having, as mentioned above, the same amino acid composition as that of elastase-1 but also from its enzymatic activity. Moreover, it did not act on TAME, a typical substrate for trypsin, and it showed only slight activity on BTEE, a typical substrate for chymotrypsin. However, its activity was about 1/5 of that of porcine elastase made by Sigma. From these, no trypsin or chymotrypsin is considered to have been contained in said human elastase.

Table 2

| | Elastin (comparative value*¹) | Casein (comparative value*²) | SA,PNA (nmol/min/mg) | TAME ($\mu$ mol/min/mg) | BTEE ($\mu$ mol/min/mg) |
|---|---|---|---|---|---|
| Human elastase | 2-15 | 34.7 | 441.2 | ⟨1 | ⟨1 |
| Porcine elastase | 100 | 30.6 | 2481.8 | ⟨1 | 4.0 |
| Bovine chymotrypsin | ±0 | 96.5 | 2.0 | ⟨1 | 29.0 |
| Bovine trypsin | ±0 | 100 | ±0 | 132.0 | ⟨1 |

*¹:  With porcine elastase taken as 100

*²:  With bovine trypsin taken as 100

EP 0 398 621 B1

(3) Preparation of anti-E1 serum

In 0.25 of physiological saline was dissolved 0.25 mg of E1, which was then emulsified with 0.25 ml of Freund's complete adjuvant. This was injected into a domestic rabbit (Japanese white) 5 times at intervals of 3 weeks, and thereby antiserum was prepared. For radioimmunoassay, a 65000-fold dilution of said antiserum was used.

(4) Preparation of $^{125}$I-labelled E1

The $^{125}$I-labelling of E1 was performed by chloramine T method. To 5 $\mu$g of E1 were added 1 mCi of Na$^{125}$I (made by Amersham) and 50 $\mu$l of 0.5 M phosphate buffer (pH 7.4). After these were mixed, 10 $\mu$l of 0.2% solution of chloramine T was added and the mixture was stirred for 30 seconds at room temperature. Then, 50 $\mu$l of 0.25% solution of sodium metabisulfite was added. After the solution was mixed, 10 $\mu$l each of 5% potassium iodide and 1% solution of bovine serum albumin were added, which was then subjected to gel filtration [Sephadex G-50, ø 0.9 x 25 cm, developing solution: 0.1 M phosphate buffer solution pH 7.4)], whereby the $^{125}$I-labelled E1 fraction was collected. To this was added the measuring buffer solution mentioned later in (7) and the mixture was allowed to stand overnight so as to bind Boc-TACK to E1. The solution was made into a 400000 dpm/ml preparation, which was then used for radioimmunoassay. The labelling rate was about 50% and the specific radioactivity was about 80 $\mu$Ci/$\mu$g.

(5) Second antibody

Immunobead-goat anti-rabbit IgG (made by Bio-Rad) was made into a 100 $\mu$g/ml preparation.

(6) Gel filtration

To human serum was added $^{125}$I-labelled E1, which was incubated for 30 minutes at 37°C. This was then subjected to gel filtration [Ultrogel AcA 44 (made by IBF Biotechnichs), ø 1.5 x 55cm, developing solution: 0.1 M phosphate buffer (pH 7.1)]. The eluate was collected in 1-ml portions and the radioactivity in each fraction was measured.

(7) Radioimmunoassay

As for measuring buffer solution, 0.01 M phosphate buffer (pH 7.4) containing 0.1 mM Boc-TACK, 0.9% sodium chloride, 0.05% sodium azide and 0.2% bovine serum albumin was used unless otherwise mentioned. One hundred $\mu$l of standard E1 solution (0.5 - 50ng/ml) or sample serum was taken into a polystyrene tube. To this were added 100 $\mu$l each of $^{125}$I-labelled E1 solution and anti-E1 serum, which was then mixed and incubated for 3 hours at 37°C. Next, 500 $\mu$l of the preparation of the second antibody was added, which was mixed and incubated for 30 minutes at 37°C. The incubated product was centrifuged (3000 rpm, 5 minutes), after which the supernatant was removed by suction and the radioactivity of the precipitate was measured.

II. Inhibition activity of Boc-TACK on the binding of serum inhibitor to $^{125}$I-labelled E1

(1) Evaluation by gel filtration

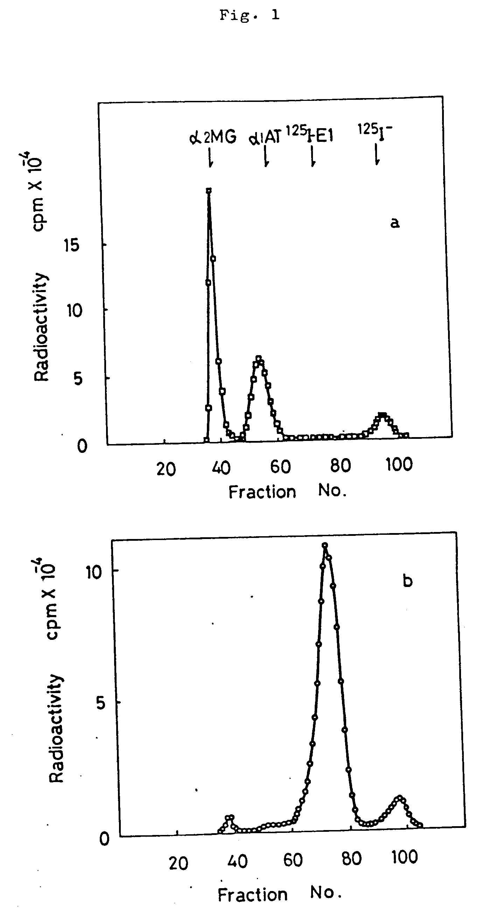

When the human serum to which $^{125}$I-labelled E1 not treated with Boc-TACK had been added was subjected to gel filtration, the $^{125}$I-labelled E1 was all eluted in the fraction of its complex containing $\alpha_1$-antitrypsin and $\alpha_2$-macroglobulin (Fig. 1-a). On the other hand, most of the $^{125}$I-labelled E1 treated with Boc-TACK was eluted in the free fraction, and none of it was found in the complex fraction (Fig. 1-b).

(2) Evaluation by an immunological procedure (radioimmunoassay)

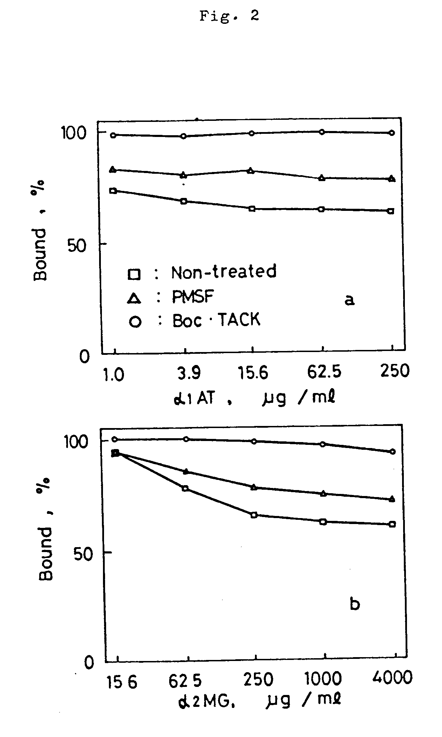

Radioimmunoassay was performed using $\alpha_1$-antitrypsin or $\alpha_2$-macroglobulin solution in place of the standard E1 solution, whereby the effect of these on the binding rate was examined. As for measuring buffer solution, one mentioned in above-mentioned (7) was used in one method (A method), and one not containing Boc-TACK was used in another method (B method), and one containing PMSF in place of Boc-TACK was used in third method (C method) (Fig. 2). In method B, the binding rate decreased gradually with increases in serum inhibitor and similar decreases were also seen in method C. On the other hand, in method A, little change was observed in the binding rate and only a slight decrease was seen with $\alpha_2$-macroglobulin in a high concentration.

## III. Measurement of human serum E1

### (1) Standard curve and dilution curve of human serum

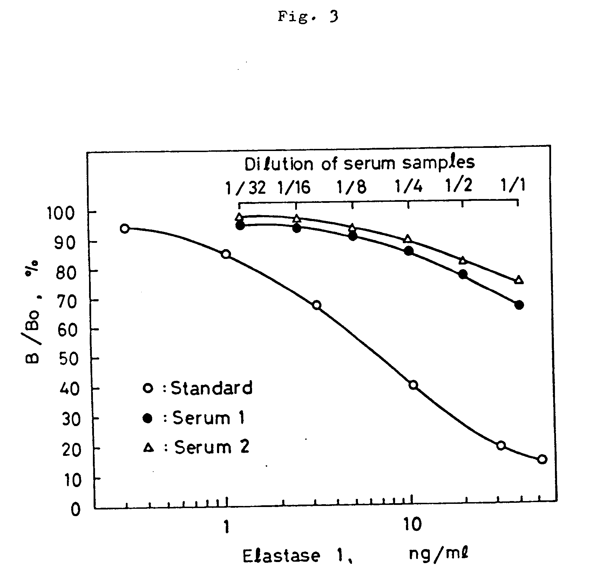

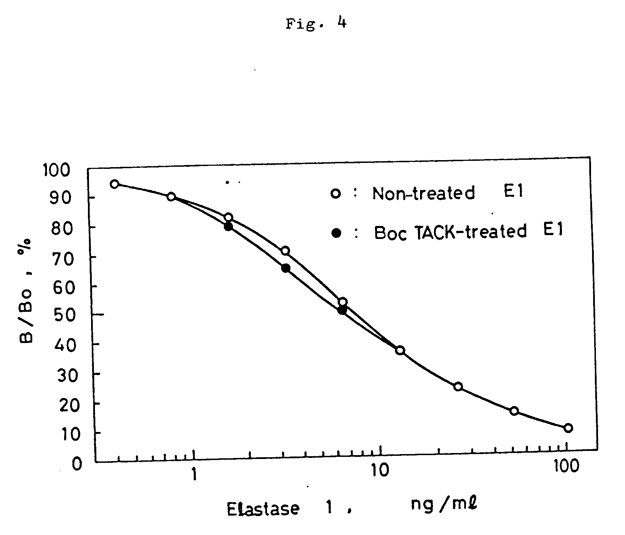

With regard to the standard curve, as shown in Fig. 3, a good curve of competitive reaction was observed within a range of 0.3 - 50 ng/ml, and the sensitivity was 0.5 ng/ml. To evaluate the effect of Boc-TACK on the immunological reaction of E1, standard curves were drawn of Boc-TACK-treated E1 and untreated E1, and then comparison was made between the two. As shown in Fig. 4, no difference was observed between the two, and even when Boc-TACK was bound to E1, it did not affect the immunological reaction of E1 in any way.

Meanwhile, the dilution curve of human serum was roughly in parallel with the standard curve (Fig. 3).

### (2) Specificity

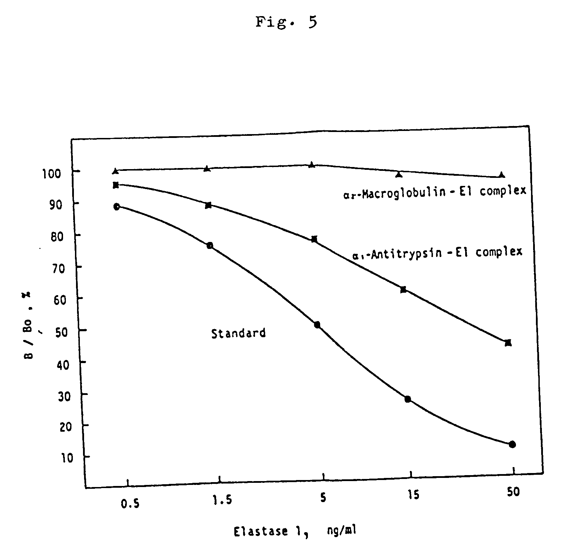

Competitive reaction curves were drawn of serum inhibitor and E1 complexes based on the results of radioimmunoassay, and cross reactivity of each complex was examined by making comparison with the standard curve of E1 (Fig. 5). Cross reactivity of $\alpha_1$-antitrypsin, $\alpha_2$-macroglobulin and E1 complexes are shown in Table 3. When cross reactivity of standard E1 is taken as 100%, the values of E1 complex containing $\alpha_1$-antitrypsin and that containing $\alpha_2$-macroglobulin are 26% and 2% or less, respectively. As shown in the results of radioimmunoassay reported in [Atsuo Murata et al.: Japanese Journal of Gastro-Enterology, 78, 1985 - 1990 (1981)], cross reactivity was observed only with E1 complex containing $\alpha_1$-antitrypsin. When acting alone, neither $\alpha_1$-antitrypsin nor $\alpha_2$-macroglobulin showed any cross reactivity.

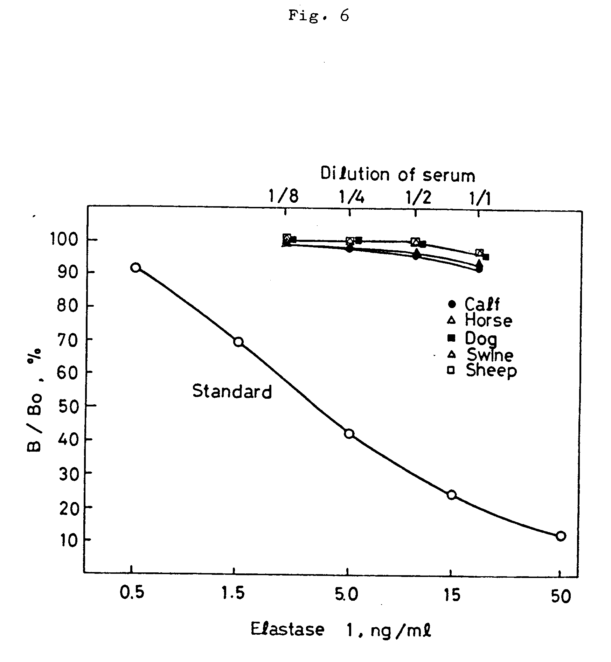

Besides, as shown in Fig. 6, no cross reactivity was seen with any of the animal sera.

## Table 3

## Cross reactivity of $\alpha_1$-antitrypsin ($\alpha_1$-AT), $\alpha_2$-macroglobulin ($\alpha_2$-MG) and E1 complexes

| Inhibitor or E1 complex with it | Cross reactivity (%) |
|---|---|
| E1-$\alpha_1$-AT | 26 |
| $\alpha_1$-AT | ⟨0.1 |
| E1-$\alpha_2$-MG | ⟨2.0 |
| $\alpha_2$-MG | ⟨0.1 |

### (3) Recovery of human serum E1

A specimen of human serum to which Boc-TACK-treated E1 had been added beforehand was measured, and thereby the recovery was calculated. A good result was obtained, with the recovery being 100.4 ± 8.9% as shown in Table 4.

Table 4

Recovery of Boc-TACK-treated E1 added to human serum

| Added amount (ng/ml) | Value of measurement (ng/ml) | Recovery (%) |
|---|---|---|
| 0 | 2.9 | |
| 0.5 | 3.6 | 106 |
| 1.5 | 4.8 | 109 |
| 5.0 | 7.8 | 99 |
| 15 | 15.4 | 86 |
| 50 | 54.0 | 102 |
| Average recovery | | 100.4±8.9 (%) |

(4) Serum E1 in healthy humans

Serum E1 was measured on 6 healthy adults. The average value was 2.38 ± 0.72 ng/ml, which was nearly in agreement with the results obtained with a radioimmunoassay kit (made by Dainabot) commercially available. (Table 5)

Table 5

Serum E1 measured in healthy humans

| Serum No. | Serum E1 (ng/ml) | |
|---|---|---|
| | Method of this invention | With the kit |
| 1 | 3.4 | 2.5 |
| 2 | 2.3 | 2.2 |
| 3 | 2.0 | 1.9 |
| 4 | 2.2 | 1.6 |
| 5 | 1.4 | 1.6 |
| 6 | 3.0 | 2.7 |
| Average | 2.38±0.72 | 2.08±0.46 |

**Claims**

**Claims for the following Contracting States : DE, GB, FR, IT, NL, SE, LI, CH, BE, AT, LU, DK**

1. An immunoassay of elastase-1, which comprises subjecting (a) labelled elastase-1 treated with Boc-TACK (Boc-Ala$_3$-CH$_2$Cl) and (b) elastase-1 to be measured to competitive reaction with anti-elastase-1 antibody.

2. An immunoassay according to claim 1, which further comprises obtaining a fraction of antibody-bound labelled elastase-1 and a fraction of free labelled elastase-1 and measuring the activity of the label in either or both of said fractions.

3. An immunoassay according to claim 1 or 2, which further comprises calculating the amount of the elastase-1 to be measured from the standard curve obtained from standard elastase-1 having a known concentration.

4. An immunoassay according to any one of claims 1 to 3, wherein said anti-elastase-1 antibody is anti-elastase-1 antiserum.

5. An immunoassay according to any one of claims 1 to 4, wherein the label is a radioisotope.

6. An immunoassay according to claim 2, wherein the antibody-bound labelled elastase-1 fraction and the free labelled elastase-1 is obtained using the double antibody method.

7. An immunoassay according to claim 6, wherein Immunobead-goat anti-rabbit IgG is used as a second antibody.

8. An immunoassay according to any one of claims 1 to 7, wherein the elastase-1 to be measured is blood elastase-1.

9. Boc-TACK-treated labelled elastase-1 obtainable by treating labelled human elastase-1 with Boc-TACK.

10. Boc-TACK-treated labelled human elastase-1 according to claim 9, wherein the label is a radioisotope.

11. Boc-TACK-treated labelled human elastase-1 according to claim 10, wherein said radioisotope is $^{125}$I.

**Claims for the following Contracting States : ES, GR**

1. An immunoassay of elastase-1, which comprises subjecting (a) labelled elastase-1 treated with Boc-TACK (Boc-Ala$_3$-CH$_2$Cl) and (b) elastase-1 to be measured to competitive reaction with anti-elastase-1 antibody.

2. An immunoassay according to claim 1, which further comprises obtaining a fraction of antibody-bound labelled elastase-1 and a fraction of free labelled elastase-1 and measuring the activity of the label in either or both of said fractions.

3. An immunoassay according to claim 1 or 2, which further comprises calculating the amount of the elastase-1 to be measured from the standard curve obtained from standard elastase-1 having a known concentration.

4. An immunoassay according to any one of claims 1 to 3, wherein said anti-elastase-1 antibody is anti-elastase-1 antiserum.

5. An immunoassay according to any one of claims 1 to 4, wherein the label is a radioisotope.

6. An immunoassay according to claim 2, wherein the antibody-bound labelled elastase-1 fraction and the free labelled elastase-1 is obtained using the double antibody method.

7. An immunoassay according to claim 6, wherein Immunobead-goat anti-rabbit IgG is used as a second antibody.

8. An immunoassay according to any one of claims 1 to 7, wherein the elastase-1 to be measured is blood elastase-1.

9. A process for the preparation of Boc-TACK-treated labelled elastase-1 comprising adding together labelled elastase-1 and Boc-TACK in a suitable buffer solution.

**Patentansprüche**

**Patentansprüche für folgende Vertragsstaaten : DE, GB, FR, IT, NL, SE, LI, CH, BE, AT, LU, DK**

1. Immuntest auf Elastase-1, welcher das Durchführen einer Verdrängungsreaktion von (a) markierter Elastase-1, die mit Boc-TACK (Boc-Ala$_3$-CH$_2$Cl) behandelt wurde, und (b) der zu messenden Elastase-1 mit Anti-Elastase-1-Antikörper umfaßt.

2. Immuntest nach Anspruch 1, der ferner das Gewinnen einer Fraktion von Antikörper gebundener markierter Elastase-1 und einer Fraktion von freier markierter Elastase-1 und die Messung der Aktivität der Markierung in einer von beiden oder beiden Fraktionen umfaßt.

3. Immuntest nach Anspruch 1 oder 2, der ferner die Berechnung der Menge der zu messenden Elastase-1 aus der Standardkurve, erhalten durch Standard-Elastase-1 mit einer bekannten Konzentration, umfaßt.

4. Immuntest nach einem der Ansprüche 1 bis 3, worin der Anti-Elastase-1-Antikörper ein Anti-Elastase-1-Antiserum ist.

5. Immuntest nach einem der Ansprüche 1 bis 4, worin die Markierung ein Radioisotop ist.

6. Immuntest nach Anspruch 2, worin die Antikörper gebundene markierte Elastase-1-Fraktion und die freie markierte Elastase-1 der Verwendung eines Doppelantikörper-Verfahrens erhalten wird.

7. Immuntest nach Anspruch 6, worin Immun-Perle-Ziege-Anti-Kaninchen-IgG als zweiter Antikörper verwendet wird.

8. Immuntest nach einem der Ansprüche 1 bis 7, worin die zu messende Elastase-1 eine Blutelastase-1 ist.

9. Boc-TACK behandelte markierte Elastase-1, erhältlich durch Behandeln markierter menschlicher Elastase-1 mit Boc-TACK.

10. Boc-TACK behandelte markierte menschliche Elastase-1 nach Anspruch 9, worin die Markierung ein Radioisotop ist.

11. Boc-TACK behandelte markierte menschliche Elastase-1 nach Anspruch 10, worin das Radioisotop $^{125}$Jod ist.

**Patentansprüche für folgende Vertragsstaaten : ES, GR**

1. Immuntest auf Elastase-1, welcher das Durchführen einer Verdrängungsreaktion von (a) markierter Elastase-1, die mit Boc-TACK (Boc-Ala$_3$-CH$_2$Cl) behandelt wurde, und (b) der zu messenden Elastase-1 mit Anti-Elastase-1-Antikörper umfaßt.

2. Immuntest nach Anspruch 1, der ferner das Gewinnen einer Fraktion von Antikörper gebundener markierter Elastase-1 und einer Fraktion von freier markierter Elastase-1 und die Messung der Aktivität der Markierung in einer von beiden oder beiden Fraktionen umfaßt.

3. Immuntest nach Anspruch 1 oder 2, der ferner die Berechnung der Menge der zu messenden Elastase-1 aus der Standardkurve, erhalten durch Standard-Elastase-1 mit einer bekannten Konzentration, umfaßt.

4. Immuntest nach einem der Ansprüche 1 bis 3, worin der Anti-Elastase-1-Antikörper ein Anti-Elastase-1-Antiserum ist.

5. Immuntest nach einem der Ansprüche 1 bis 4, worin die Markierung ein Radioisotop ist.

6. Immuntest nach Anspruch 2, worin die Antikörper gebundene markierte Elastase-1-Fraktion und die freie markierte Elastase-1 der Verwendung eines Doppelantikörper-Verfahrens erhalten wird.

7. Immuntest nach Anspruch 6, worin Immun-Perle-Ziege-Anti-Kaninchen-IgG als zweiter Antikörper verwendet wird.

8. Immuntest nach einem der Ansprüche 1 bis 7, worin die zu messende Elastase-1 eine Blutelastase-1 ist.

9. Verfahren zur Herstellung Boc-TACK behandelter, markierter Elastase-1, umfassend die Zugabe von markierter Elastase-1 zusammen mit Boc-TACK in eine geeignete Pufferlösung.

**Revendications**

**Revendications pour les Etats contractants suivants : DE, GB, FR, IT, NL, SE, LI, CH, BE, AT, LU, DK**

1. Analyse immunologique de l'élastase-1, qui comprend la réalisation d'une réaction compétitive de (a) l'élastase-1 marquée, traitée par le Boc-TACK (Boc-Ala$_3$-CH$_2$Cl) et (b) l'élastase-1 à mesurer, avec un anticorps anti-élastase-1.

2. Analyse immunologique suivant la revendication 1, qui comprend, de plus, l'obtention d'une fraction d'élastase-1 marquée, fixée à l'anticorps et d'une fraction d'élastase-1 marquée, libre et la mesure de l'activité du marqueur dans une ou les deux fractions.

3. Analyse immunologique suivant la revendication 1 ou 2, qui comprend, de plus, le calcul de la quantité d'élastase-1 à mesurer sur la courbe standard obtenue à partir d'élastase-1 standard ayant une concentration connue.

4. Analyse immunologique suivant l'une quelconque des revendications 1 à 3, dans lequel l'anticorps anti-élastase-1 est un antisérum anti-élastase-1.

5. Analyse immunologique suivant l'une quelconque des revendications 1 à 4, dans lequel le marqueur est un radio-isotope.

6. Analyse immunologique suivant la revendication 2, dans lequel la fraction d'élastase-1 marquée, fixée à l'anticorps et l'élastase-1 marquée, libre sont obtenues par le procédé à double anticorps.

7. Analyse immunologique suivant la revendication 6, dans lequel l'IgG anti-lapin, de chèvre, sur perles est utilisé comme deuxième anticorps.

8. Analyse immunologique suivant l'une quelconque des revendications 1 à 7, dans lequel l'élastase-1 à mesurer est l'élastase-1 sanguine.

9. Elastase-1 marquée, traitée par le Boc-TACK, pouvant être obtenue par traitement d'élastase-1 humaine, marquée avec du Boc-TACK.

10. Elastase-1 humaine, marquée, traitée par le Boc-TACK suivant la revendication 9, dans laquelle le marqueur est un radio-isotope.

11. Elastase-1 humaine, marquée, traitée par le Boc-TACK suivant la revendication 10, dans laquelle le radio-isotope est le $^{125}$I.

**Revendications pour les Etats contractants suivants : ES, GR**

1. Analyse immunologique de l'élastase-1, qui comprend la réalisation d'une réaction compétitive de (a) l'élastase-1 marquée, traitée par le Boc-TACK (Boc-Ala$_3$-CH$_2$Cl) et (b) l'élastase-1 à mesurer, avec un anticorps anti-élastase-1.

2. Analyse immunologique suivant la revendication 1, qui comprend, de plus, l'obtention d'une fraction d'élastase-1 marquée, fixée à l'anticorps et d'une fraction d'élastase-1 marquée, libre et la mesure de l'activité du marqueur dans une ou les deux fractions.

3. Analyse immunologique suivant la revendication 1 ou 2, qui comprend, de plus, la calcul de la quantité d'élastase-1 à mesurer sur la courbe standard obtenue à partir d'élastase-1 standard ayant une concentration connue.

4. Analyse immunologique suivant l'une quelconque des revendications 1 à 3, dans lequel l'anticorps anti-élastase-1 est un antisérum anti-élastase-1.

5. Analyse immunologique suivant l'une quelconque des revendications 1 à 4, dans lequel le marqueur est un radio-isotope.

6. Analyse immunologique suivant la revendication 2, dans lequel la fraction d'élastase-1 marquée, fixée à l'anticorps et l'élastase-1 marquée, libre sont obtenues par le procédé à double anticorps.

7. Analyse immunologique suivant la revendication 6, dans lequel l'IgG anti-lapin, de chèvre, sur perles est utilisé comme deuxième anticorps.

8. Analyse immunologique suivant l'une quelconque des revendications 1 à 7, dans lequel l'élastase-1 à mesurer est l'élastase-1 sanguine.

9. Procédé de préparation de l'élastase-1 humaine, marquée, traitée par le Boc-TACK, comprenant le mélange de l'élastase-1 marquée et du Boc-TACK dans une solution tampon appropriée.

Fig. 1

Fig. 2

Fig. 3

Fig. 4

Fig. 5

Fig. 6